# EUROPEAN PATENT SPECIFICATION

(11) **EP 3 099 658 B1**
(45) Date of publication and mention of the grant of the patent: **28.02.2018**
(21) Application number: 15702708.7
(22) Date of filing: 27.01.2015
(51) Int. Cl.: C07C 37/14

(54) **PROCESS OF PRODUCTION OF 2,3,6-TRIMETHYLPHENOL**
VERFAHREN ZUR HERSTELLUNG VON 2,3,6-TRIMETHYLPHENOL
PROCÉDÉ DE PRODUCTION DE 2,3,6-TRIMÉTHYLPHÉNOL

(30) Priority: 27.01.2014 EP 14152691
(43) Date of publication of application: 07.12.2016
(73) Proprietor: DSM IP Assets B.V., 6411 TE Heerlen (NL)
(72) Inventor: BONRATH, Werner, CH-4303 Kaiseraugst (CH); LETINOIS, Ulla, CH-4303 Kaiseraugst (CH); NETSCHER, Thomas, CH-4303 Kaiseraugst (CH)
(74) Representative: Dux, Roland
(86) International application number: PCT/EP2015/051599
(87) International publication number: WO 2015/110654

(56) References cited:
- HUGUET, NURIA ET AL: "Intermolecular Gold(I)-Catalyzed Cyclization of Furans with Alkynes: Formation of Phenols and Indenes", CHEMISTRY - A EUROPEAN JOURNAL, vol. 19, no. 21, 4 April 2013 (2013-04-04) , pages 6581-6585, XP002726348, ISSN: 0947-6539, DOI: 10.1002/CHEM.201300646
- Tomoharu Oku ET AL: "Continuous Chemoselective Methylation ofm-Cresol and Phenol with Supercritical Methanol over Solid Acid and Base Metal Oxide Catalysts", Advanced Synthesis & Catalysis, vol. 347, no. 11-13, 1 October 2005 (2005-10-01), pages 1553-1557, XP055382382, DE ISSN: 1615-4150, DOI: 10.1002/adsc.200505191

## Description

The present invention relates to a new method to produce 2,3,6-trimethylphenol (2,3,6-TMP).

2,3,6-trimethylphenol, can be used for example as an intermediate in the production of vitamin E.

2,3,6-TMP is the compound of formula (I)

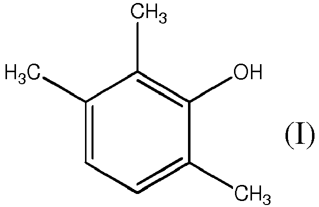

Due to the importance of the 2,3,6-TMP new methods of its production are always of interest.
It is known from prior art to produce 2,3,6-TMP from for example 2,5-dimethylphenol by methylation. 2,5-Dimethylphenol is traditionally extracted from coal tar, which is a non-renewable source.
Therefore there is always a need for a method of production of 2,3,6-TMP, wherein the starting material is obtained from a renewable source.

Now, it was found that it is possible to produce 2,3,6-TMP in a one-step reaction from 2,5-dimethylfuran, which is the compound of formula (II)

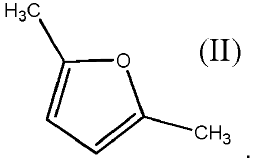

2,5-Dimethylfuran is obtained from a renewable source (cellulose).

This process is known from the prior art (i.e. Y. Román-Leshkov, C. J. Barrett, Z. Y. Liu, J. A. Dumesic, Nature 2007, 447, 982-985).

Tomoharu Oku et al., Adv. Synth. Catal. 2005, 347, 1553-1557, disclose a process for producing 2,3,6-TMP from meta-cresol and phenol by catalytic methylation with methanol. It is known that fructose can be converted into 2,5-dimethylfuran in a catalytic biomass-to-liquid process. Fructose is obtainable from glucose, a building block in cellulose.
It was found that 2,5-dimethylfuran can be reacted with propyne or propyne derivatives in the presence of a catalyst to obtain 2,3,6-TMP.
Therefore the present invention is related to the following process (A): 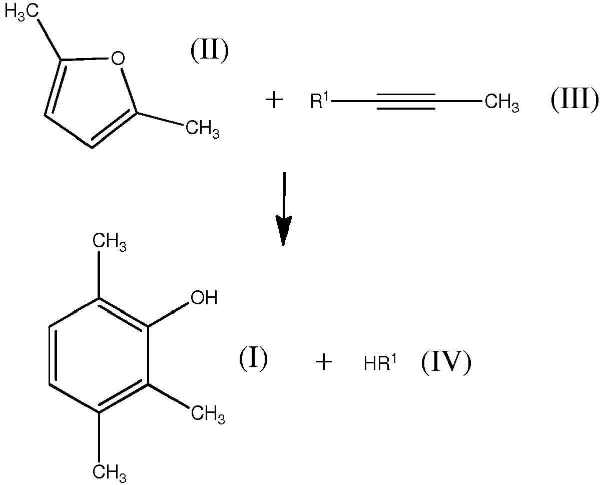 wherein R¹ is H or Si(CH₃)₃ and which is carried out in the presence of at least one catalyst.

Preferably the catalyst used in the processes as described above the catalyst is an Au(I) complex.

Therefore the present invention also relates to process (B), which process (A), wherein the catalyst is an Au(I) complex.

Especially the following Au(I)-complexes are used as catalysts for the process according to the present invention:

Y-Au(I)-Z (V),

wherein
Z is an anion, which is selected from the group consisting of [BX₄]⁻, [PX₆]⁻, [SbF₆]⁻, [ClO₄]⁻, CF₃COO⁻, sulfonates, tetra(3,5-bis(trifluoromethyl)phenyl)borate (BAr_{F}⁻), tetraphenylborate, and the following anion of formula (VI) 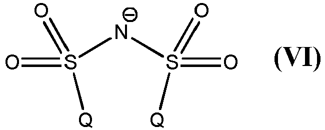
   wherein Q represents a phenyl or a C₁₋₈-alkyl which preferably is substituted by at least one substituent chosen from the group consisting of F, Cl and NO₂, and
   X is a halogen atom, especially F and Cl, and
Y is an organic ligand.

Therefore the present invention also relates to process (C), which is the process (A) or (B), wherein at least one Au(I) complex of the following compound of formula (V) is used

Y-Au(I)-Z (V),

wherein
Z is an anion, which is selected from the group consisting of [BX₄]⁻, [PX₆]⁻, [SbF₆]⁻, [ClO₄]⁻, CF₃COO⁻, sulfonates, tetra(3,5-bis(trifluoromethyl)phenyl)borate (BAr_{F}⁻), tetraphenylborate, and the following anion of formula (VI) 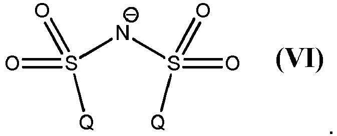
   wherein Q represents a phenyl or a C₁₋₈-alkyl group which preferably is substituted by at least one substituent chosen from the group consisting of F, Cl and NO₂, and
   X is a halogen atom, especially F and Cl, and
Y is an organic ligand.

Preferably Y is an organic ligand selected from the group consisting of following ligands (Y¹) to (Y¹⁰): 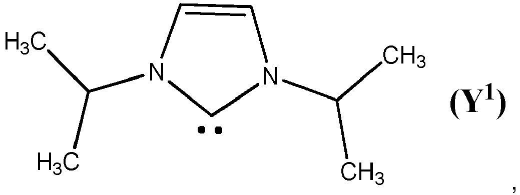 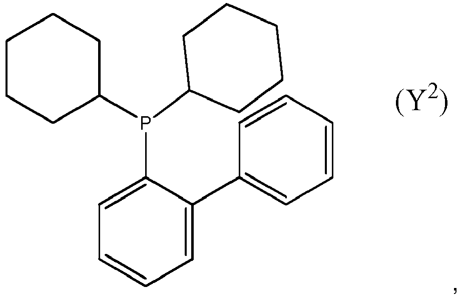 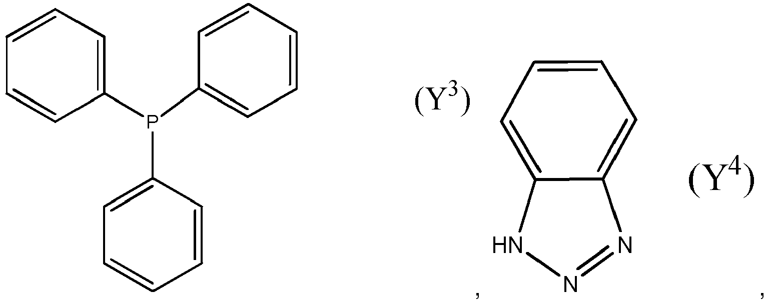 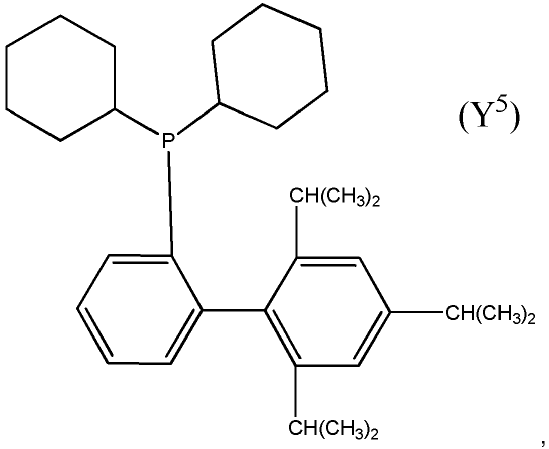 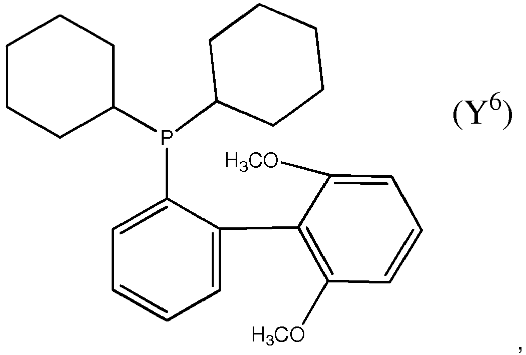 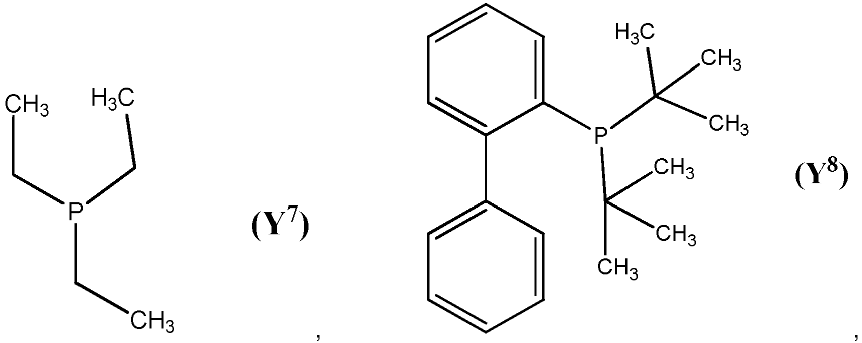 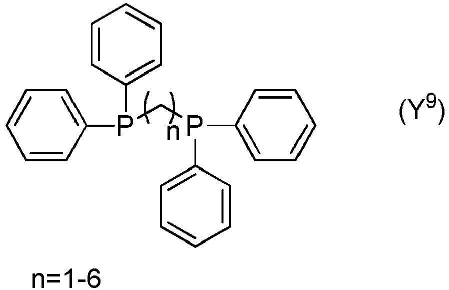 and

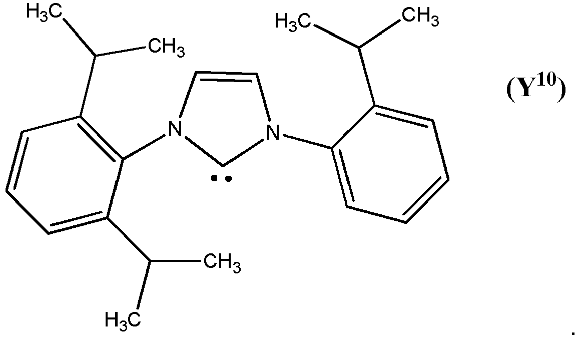

Therefore the present invention also relates to process (C'), which is the process (C), wherein the organic ligand Y of Au(I) complex of formula (V) is selected from the group consisting of following ligands (Y¹) to (Y¹⁰): 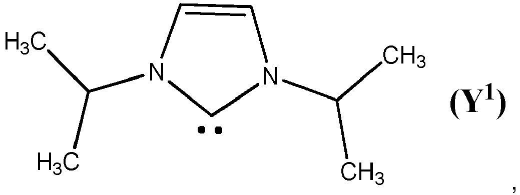 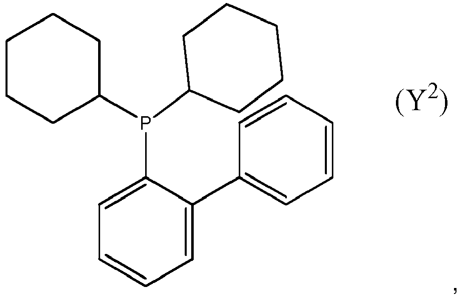 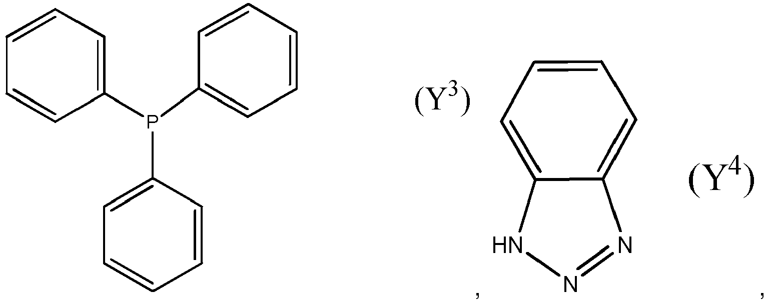 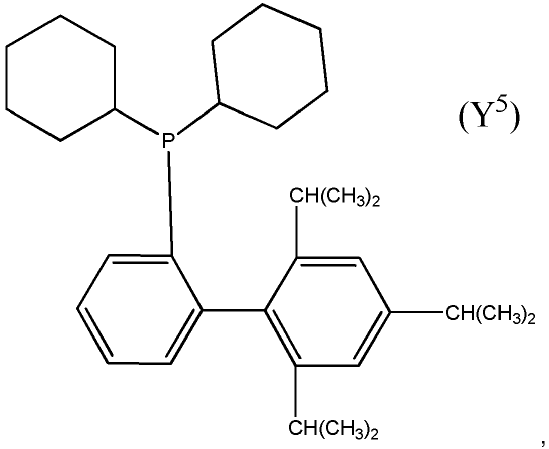 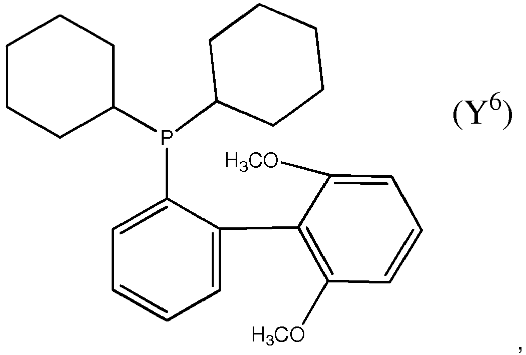 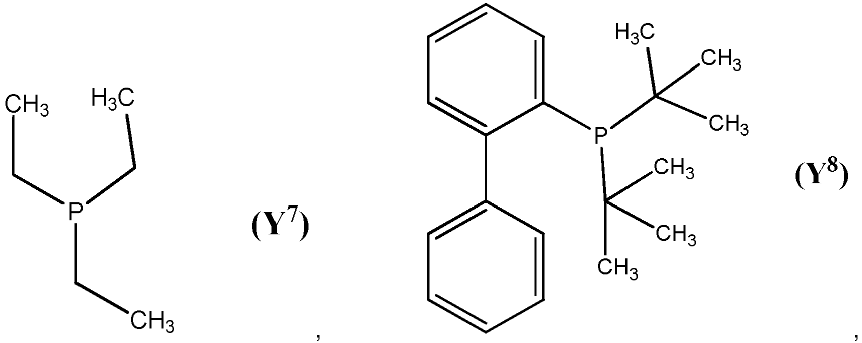 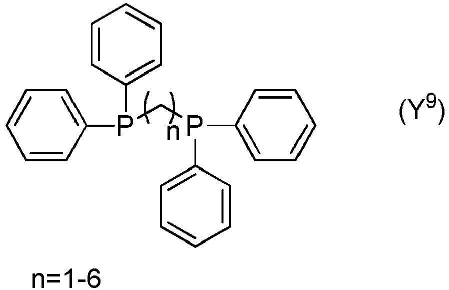 and

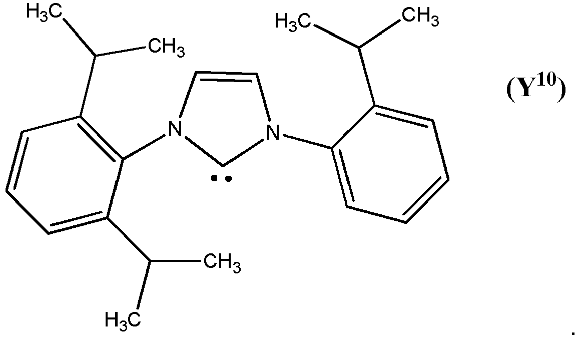

Preferably Z is an anion selected from the group consisting of following anions [BF₄]⁻, [PF₆]⁻, [SbF₆]⁻, [ClO₄]⁻, CF₃COO⁻, sulfonates (such as triflate CF₃SO₃⁻), tetra(3,5-bis(trifluoromethyl)phenyl)borate (BAr_{F}⁻), tetraphenylborate, and the following anion of formula (VI')

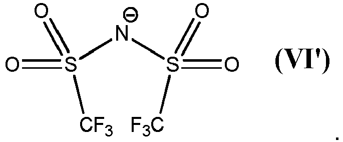

Therefore the present invention also relates to process (C"), which is the process (C) or (C'), wherein the anion Z is selected from the group consisting of following anions [BF₄]⁻, [PF₆]⁻, [SbF₆]⁻, [ClO₄]⁻, CF₃COO⁻, sulfonates, (such as triflate CF₃SO₃⁻), tetra(3,5-bis(trifluoromethyl)phenyl)borate (BAr_{F}⁻), tetraphenylborate, and the following anion of formula (VI')

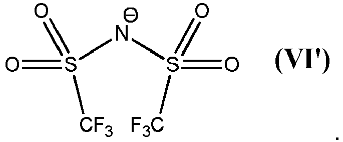

The catalyst (the Au(I) complex) can be added to the reaction mixture in the form, which is defined by formula (I), but it is also possible that the Au(I)-complex is formed in situ in the reaction mixture (before the starting material is added or after the starting material is added).

For example it is possible to add the organic ligand in the form of a salt (such as for example a chloride: Y-Au(I)Cl) and the anion in form of a metal salt (such for example a silver salt Ag(I)Z). The Au(I) complex is then formed in situ and the resulting metal salt (for example AgCl) does not interfere negatively.

Therefore the present invention relates to a process (D), which is the process (C), (C') or (C"), wherein the Au(I) complex is added to the reaction mixture as such.

Furthermore the present invention relates to a process (E), which is the process (C), (C') or (C"), wherein the Au(I) complex is formed in situ in the reaction mixture.

Preferred Au(I) complexes of formula (V) are the following one. 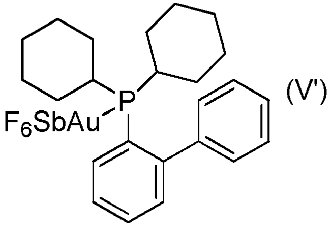 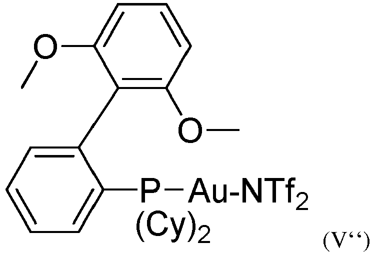 and 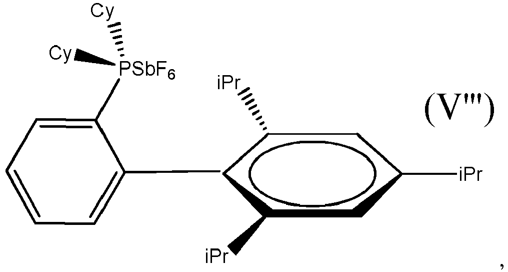 wherein Cy is cyclohexyl, iPr is isopropyl and Tf is triflate.

When a propyne derivate is used (instead of propyne), then R¹ is chosen in a way that this derivative is easier handable than propyne and wherein R¹ is forming a reaction product which can be removed easily from the reaction mixture.
In the case of R₁ = Si(CH₃)₃, then the compound of formula (III) is liquid. Therefore it is also possible to use other propyne derivatives than the one with the Si(CH₃)₃ group, when they have the properties as described above.

Usually the Au(I) complex is present in an amount, wherein the substrate (compound of formula (I)) to catalyst ratio is 2 : 1 to 10000 : 1, preferred are 10 : 1 to 3000 : 1. The ratio is weight-based.

Therefore the present invention also relates to a process (F), which is the process (A), (B), (C); (C'); (C"), (D) or (E), wherein the substrate to catalyst ratio is 2 : 1 to 10000 : 1, preferred are 10 : 1 to 3000 : 1.

A further embodiment is the process according to the present invention wherein benzonitrile is added to the reaction mixture at the start of the process. Usually it is added in an equimolar amount in regard to the catalyst.

Therefore the present invention also relates to a process (G), which is the process (A), (B), (C), (C'), (C"), (D), (E) or (F), wherein benzonitrile is added to the reaction mixture.

Therefore the present invention also relates to a process (G'), which is the process (G), wherein benzonitrile is added to the reaction mixture in an equimolar amount in regard to the catalyst.

The process according to the present invention is usually carried out under normal pressure.

Therefore the present invention also relates to a process (H), which is the process (A), (B), (C), (C'), (C"), (D), (E), (F), (G) or (G'), wherein the process is carried out under normal pressure.

The reaction temperature of the process according to the present invention is usually between 10 - 50 °C. Preferably between 15 - 30 °C.

Therefore the present invention also relates to a process (I), which is the process (A), (B), (C), (C'), (C"), (D), (E), (F), (G), (G') or (H), wherein the process is carried out between 10 - 50 °C, preferably between 15 - 30 °C.

The reaction is usually carried out in an inert solvent (or mixture of solvents). Preferably the solvent (or the mixture of solvents) has a neutral or acidic pH value. Preferred solvents are dichloromethane, 1,2-dichloroethane, trifluoroethanol, chloroform, toluene, ethyl acetate, cyclohexanone, acetone.
More preferred are dichloromethane, trifluoroethanol and 1,2-dichloroethane as well as a mixture of dichloromethane with 5 vol% of trifluoroethanol.
Furthermore it is also possible to use an ionic liquid as a solvent. Very suitable ionic liquids are the following ones (IL¹, IL² and IL³):

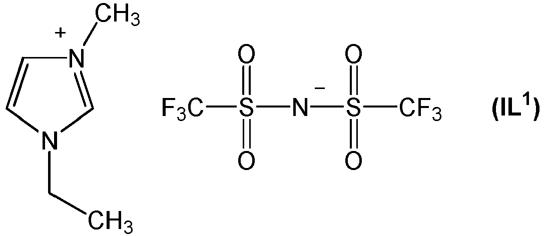

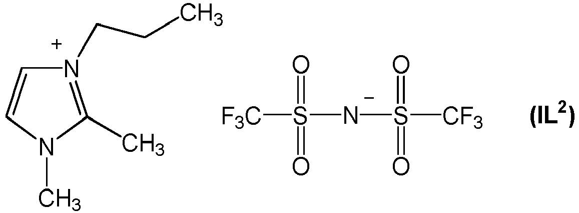

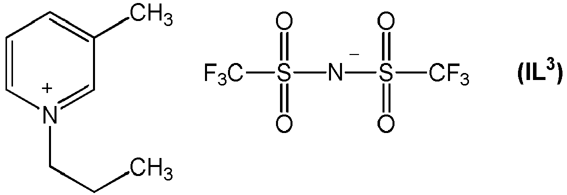

Therefore the present invention also relates to a process (K), which is the process (A), (B), (C), (C'), (C"), (D), (E), (F), (G), (G'), (H) or (I), wherein the process is carried in an inert solvent (or mixture of solvents).

Therefore the present invention also relates to a process (K'), which is the process (K), wherein the solvent (or the mixture of solvents) has a neutral or acidic pH value.

Therefore the present invention also relates to a process (K"), which is the process (K) or (K'), wherein the solvent is chosen from the group consisting of dichloromethane, 1,2-dichloroethane, trifluoroethanol, chloroform, toluene, ethyl acetate, cyclohexanone, acetone.

Therefore the present invention also relates to a process (K'''), which is the process (K), (K') or (K"), wherein the solvent is chosen from the group consisting of dichloromethane, trifluoroethanol and 1,2-dichloroethane as well as a mixture of dichloromethane with 5 vol% of trifluoroethanol.

Therefore the present invention also relates to a process (K""), which is the process (K), (K') or (K"), wherein the solvent is an ionic liquid (or a mixture of ionic liquis).

Therefore the present invention also relates to a process (K""'), which is the process (K""), wherein the ionic liquids are those of formula (IL¹), (IL²) and/or (IL³). The following examples serve to illustrate the invention. All percentages and parts (if not otherwise stated) are related to the weight and the temperature is given in °C.

### Examples

### Example 1

To a 2 mL glass bottle, fitted with a septum, a magnetic stirring bar and argon supply are added 11.95 mg of the following formula 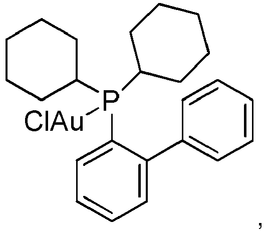 which is Y²-Au(I)Cl (20 µmol, 2 mol%), 30.66 mg AgSbF₆ (20 µmol, 2 mol%) 500 µL 2,5-dimethylfuran (1 mmol, 1 equiv.) and suspended in 1 mL dichloromethane. Then 144.2 µL trimethylsilylpropyne (1 mmol, 1 equiv.) were added by syringe. The reaction mixture was stirred for five days at 23°C. A sample was taken out by syringe and analysed. 2,3,6-trimethylphenol was obtained in 56% yield relative to 2,5-dimethylfuran.

### Example 2

A three-necked round bottom flask, fitted with argon supply, a thermometer and a magnetic stirring bar was charged with 70 mL dichloroethane. A saturated solution of propyne was prepared by bubbling the gas into the solvent. Then 356 mg of the gold complex of Y²-Au(I)Cl (600 µmol, 2 mol%), 210.5 mg AgSbF₆ (600 µmol, 2 mol%) and 10 mL 1,2-dichloroethane were added. 2,5-Dimethylfuran (3.22 ml, 30 mol, 1 equiv.) was added via syringe. The mixture was stirred at 23°C for 20 hours under continuous bubbling of propyne.

The reaction mixture was concentrated under reduced pressure. The desired product, 2,3,6-trimethylphenol was obtained in 82% (GC area%).

## Claims

1. A process for producing the compound of formula (I) 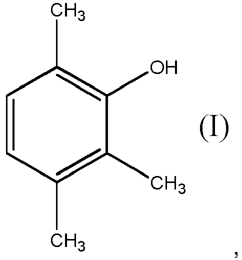 wherein the compound of formula (II) 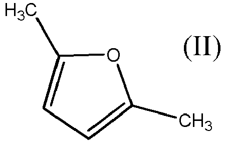 is reacted with a compound of formula (III) 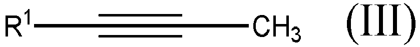 wherein R¹ is H or Si(CH₃)₃.
in the presence of at least catalyst.

2. Process according to claim 1, wherein the catalyst is an Au(I) complex..

3. Process according to claim 1 or 2, wherein at least one catalyst of formula (V)
Y-Au(I)-Z (V),
wherein
Z is an anion, which is selected from the group consisting of [BX₄]⁻, [PX₆]⁻, [SbF₆]⁻, [ClO₄]⁻, CF₃COO⁻, sulfonates, tetra(3,5-bis(trifluoromethyl)phenyl)borate (BAr_{F}⁻), tetraphenylborate, and the following anion of formula (VI) 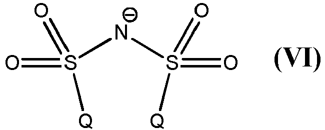
wherein Q represents a phenyl or a C₁₋₈-alkyl which preferably is substituted by at least one substituent chosen from the group consisting of F, Cl and NO₂, and
X is a halogen atom, especially F and Cl, and
Y is an organic ligand,
is used.

4. Process according to claim 3, wherein Y is an organic ligand selected from the group consisting of following ligands (Y¹) to (Y¹⁰): 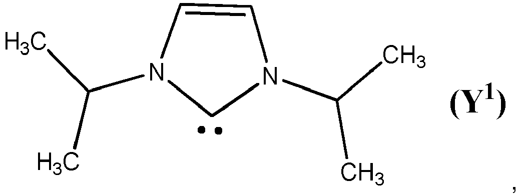 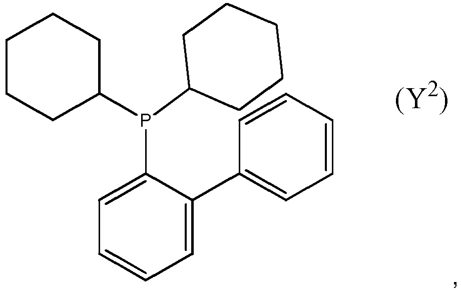 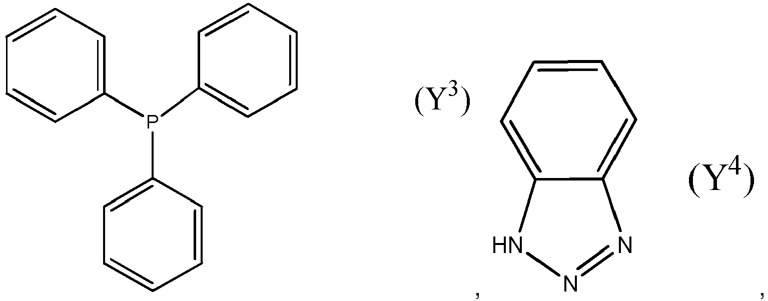 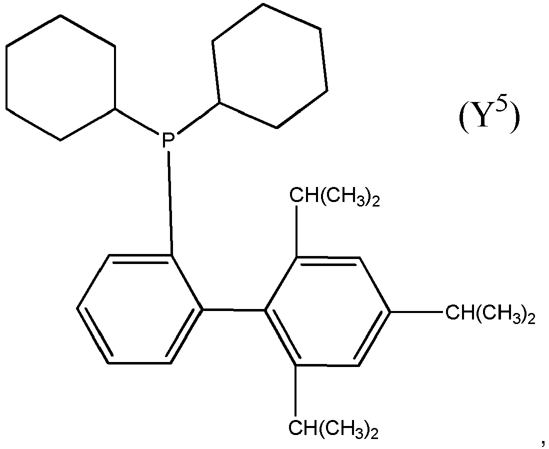 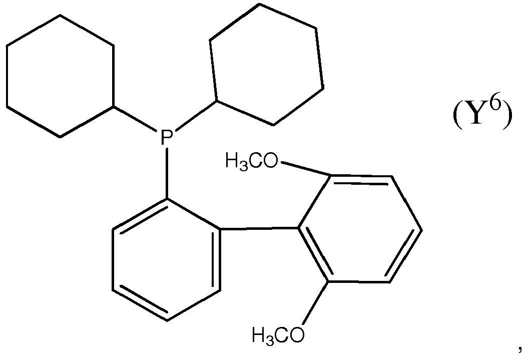 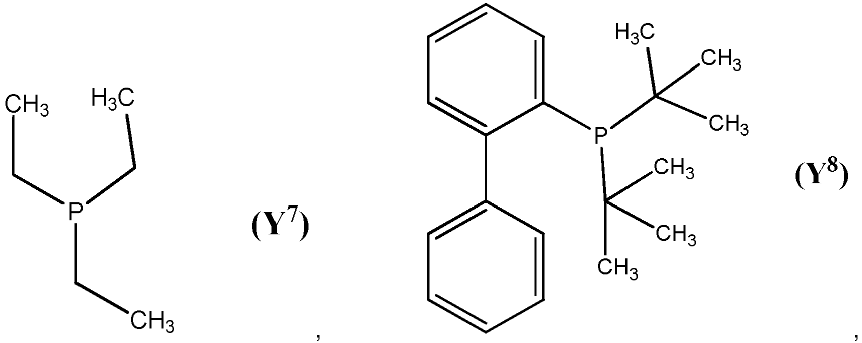 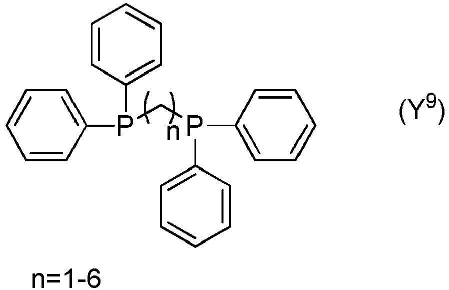 and

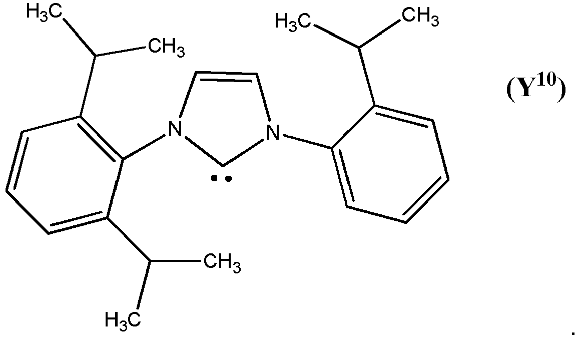

5. Process according to claim 3 and 4, wherein Z is an anion selected from the group consisting of following anions [BF₄]⁻, [PF₆]⁻, [SbF₆]⁻, [ClO₄]⁻, CF₃COO⁻, sulfonates (such as triflate), tetra(3,5-bis(trifluoromethyl)phenyl)borate (BAr_{F}⁻), tetraphenylborate, and the following anion of formula (VI')

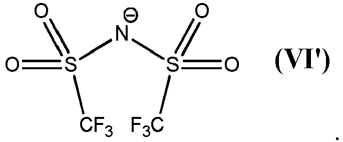

6. A process according to any of claims 3 - 5, wherein the Au(I) complex is added to reaction mixture as such.

7. Process according to any of claims 3 - 5, wherein the Au(I) complex is formed in situ in the reaction mixture.

8. Process according to any of claims 1 - 7, wherein the substrate to catalyst ratio is 2 :1 to 10000 : 1, preferred are 10 : 1 to 3000: 1.

## Patentansprüche

1. Verfahren zur Herstellung der Verbindung der Formel (I) 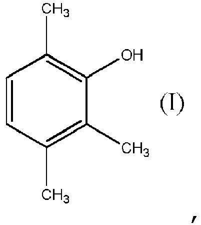 bei dem man die Verbindung der Formel (II) 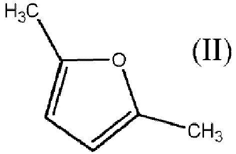 in Gegenwart von mindestens Katalysator mit einer Verbindung der Formel (III) 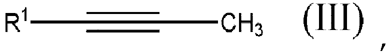 wobei R¹ für H oder Si(CH₃)₃ steht, umsetzt.

2. Verfahren nach Anspruch 1, bei dem es sich bei dem Katalysator um einen Au(I)-Komplex handelt.

3. Verfahren nach Anspruch 1 oder 2, bei dem mindestens ein Katalysator der Formel (V)
Y-Au(I)-Z (V)
wobei
Z für ein Anion steht, das aus der Gruppe bestehend aus [BX₄]⁻, [PX₆]⁻, [SbF₆]⁻, [ClO₄]⁻, CF₃COO⁻, Sulfonaten, Tetra (3,5-bis(trifluormethyl)-phenyl)borat (BAr_{F}⁻), Tetraphenylborat und dem folgenden Anion der Formel (VI) ausgewählt ist: 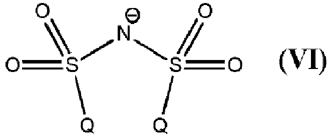
wobei Q für ein Phenyl oder ein C₁₋₈-Alkyl, das vorzugsweise durch mindestens einen Substituenten aus der Gruppe bestehend aus F, Cl und NO₂ substituiert ist, steht und
X für ein Halogenatom, speziell F und Cl, steht, und
Y für einen organischen Liganden steht,
verwendet wird.

4. Verfahren nach Anspruch 3, bei dem Y für einen organischen Liganden aus der Gruppe bestehend aus den folgenden Liganden (Y¹) bis (Y¹⁰) steht: 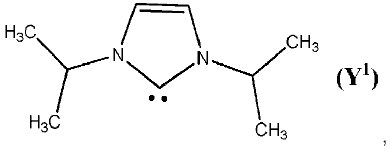 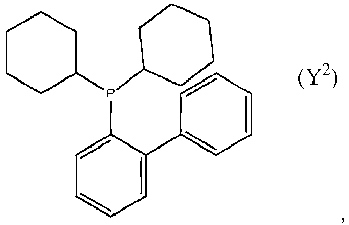 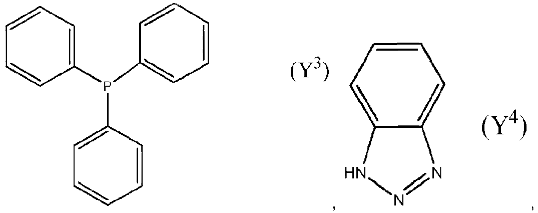 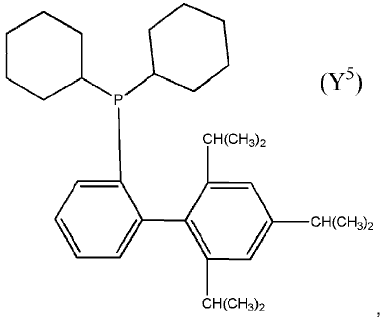 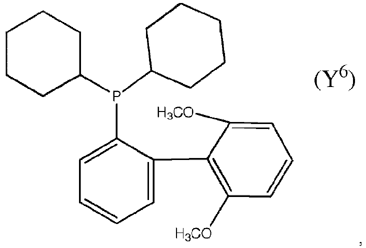 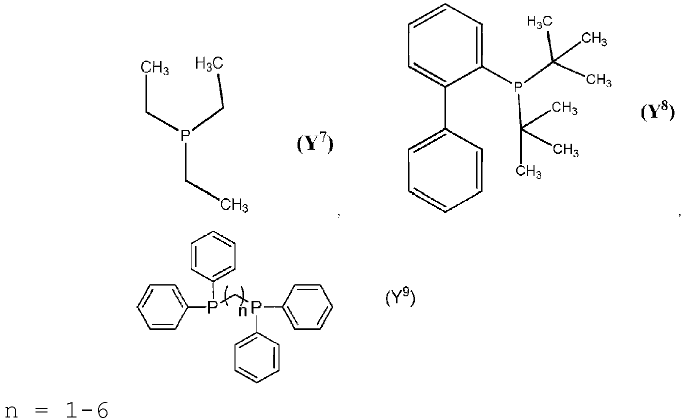 und

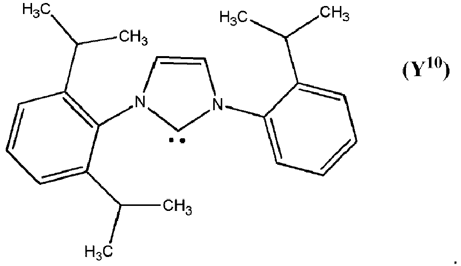

5. Verfahren nach Anspruch 3 und 4, bei dem Z für ein Anion aus der Gruppe bestehend aus den folgenden Anionen: [BF₄]⁻, [PF₆]⁻, [SbF₆]⁻, [ClO₄]⁻, CF₃COO⁻, Sulfonaten (wie Triflat), Tetra(3,5-bis(trifluormethyl)phenyl)borat (BAr_{F}⁻), Tetraphenylborat und dem folgenden Anion der Formel (VI') ausgewählt ist:

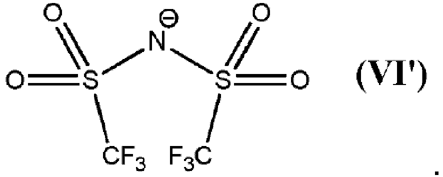

6. Verfahren nach einem der Ansprüche 3-5, bei dem der Au(I)-Komplex als solcher zur Reaktionsmischung gegeben wird.

7. Verfahren nach einem der Ansprüche 3-5, bei dem der Au(I)-Komplex in der Reaktionsmischung in situ gebildet wird.

8. Verfahren nach einem der Ansprüche 1-7, bei dem das Verhältnis von Substrat zu Katalysator 2:1 bis 10.000:1, vorzugsweise 10:1 bis 3000:1, beträgt.

## Revendications

1. Procédé de production du composé de formule (I) 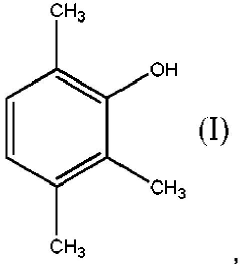 dans lequel le composé de formule (II) 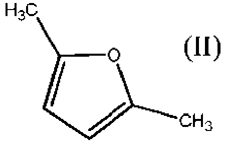 réagit avec un composé de formule (III) 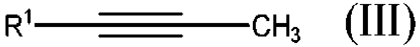 dans laquelle R¹ est H ou Si(CH₃)₃, en présence d'au moins un catalyseur.

2. Procédé selon la revendication 1, dans lequel le catalyseur est un complexe de Au(I).

3. Procédé selon la revendication 1 ou 2, dans lequel au moins un catalyseur de formule (V)
Y-Au(I)-Z (V),
dans laquelle
Z est an anion, qui est choisi dans le groupe constitué de [BX₄]⁻, [PX₆]⁻, [SbF₆]⁻, [ClO₄]⁻, CF₃COO⁻, sulfonates, tétra(3,5-bis(trifluorométhyl)phényl)borate (BAr_{F})⁻, tétraphénylborate, et l'anion suivant de formule (VI) 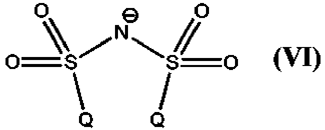
dans laquelle Q représente un phényle ou un alkyle en C₁₋₈ qui est, de préférence, substitué par au moins un substituant choisi dans le groupe constitué de F, Cl et NO₂, et
X est un atome d'halogène, en particulier F et Cl, et
Y est un ligand organique,
est utilisé.

4. Procédé selon la revendication 3, dans lequel Y est un ligand organique choisi dans le groupe constitué des ligands suivants (Y¹) à (Y¹⁰) : 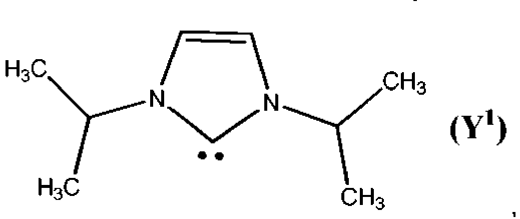 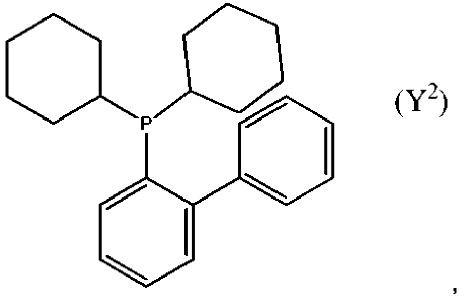 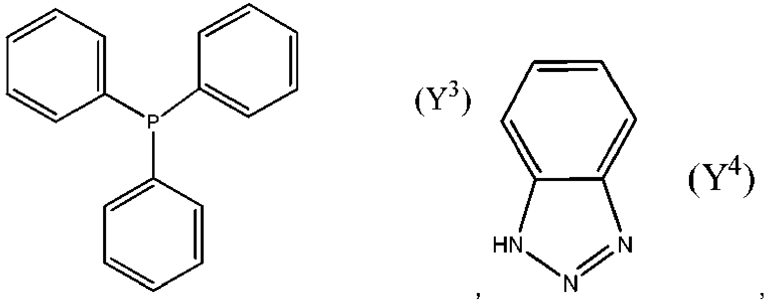 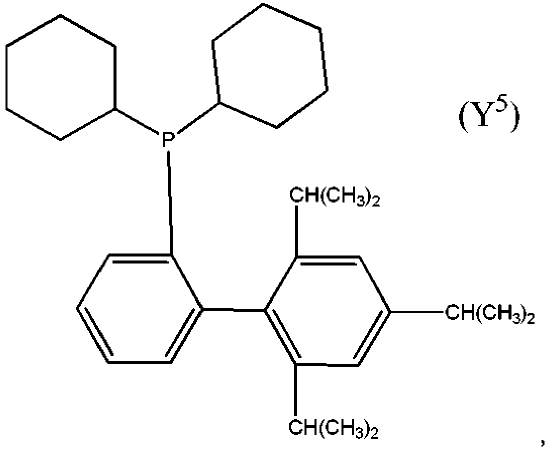 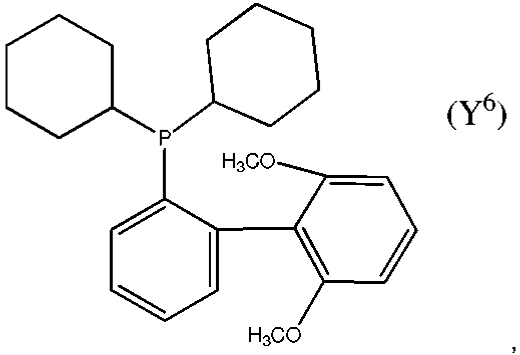 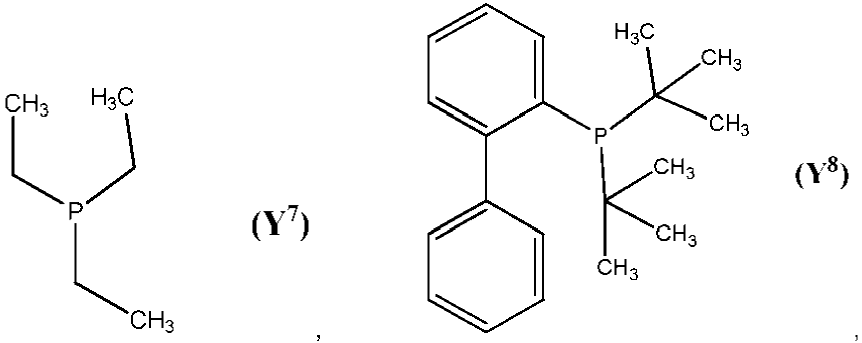 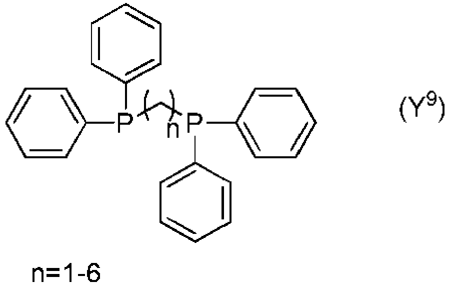 et

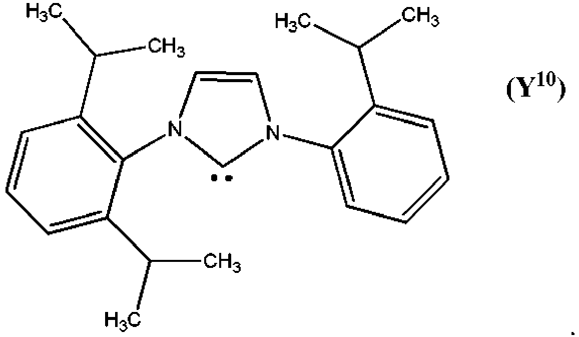

5. Procédé selon la revendication 3 et 4, dans lequel Z est un anion choisi dans le groupe constitué des anions suivants : [BF₄]⁻, [PF₆]⁻, [SbF₆]⁻, [ClO₄]⁻, CF₃COO⁻, des sulfonates (tels que triflate), tétra(3,5-bis (trifluorométhyl)phényl)borate (BAr_{F}⁻), tétraphénylborate, et l'anion de formule (VI') suivant

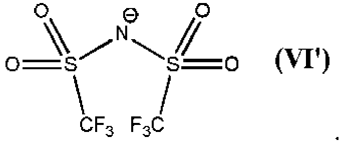

6. Procédé selon l'une quelconque des revendications 3 à 5, dans lequel le complexe de Au(I) est ajouté au mélange de réaction tel quel.

7. Procédé selon l'une quelconque des revendications 3 à 5, dans lequel le complexe de Au(I) est formé in situ dans le mélange de réaction.

8. Procédé selon l'une quelconque des revendications 1 à 7, dans lequel le rapport du substrat au catalyseur est de 2:1 à 10000:1, de préférence de 10:1 à 3000:1.
